Europäisches Patentamt

**European Patent Office**

**Office européen des brevets**

(11) Publication number: **0 115 656**
**A1**

## (12) EUROPEAN PATENT APPLICATION

(21) Application number: **83201848.5**

(22) Date of filing: **29.12.83**

(51) Int. Cl.³: **A 61 F 9/06**, G 02 B 5/22,
G 02 C 7/10

(30) Priority: **05.01.83 GB 8300149**

(43) Date of publication of application: **15.08.84**
**Bulletin 84/33**

(84) Designated Contracting States: **AT BE CH DE FR GB IT
LI LU NL SE**

(71) Applicant: **White, Graham Edwin, 105 Button Hill,
Ecclesall Sheffield S11 9HG (GB)**
Applicant: **THE UNIVERSITY OF SHEFFIELD, Western
Bank, Sheffield, S10 (GB)**

(72) Inventor: **White, Graham Edwin, 105 Button Hill,
Ecclesall Sheffield S11 9HG (GB)**

(74) Representative: **Houghton, David et al, Hulse & Co.
Cavendish Buildings West Street, Sheffield, S1 1ZZ (GB)**

(54) **Eye protection means.**

(57) The application relates to eye protection means (1), particularly for industrial application such as would be used in welding and in hot metal and glass applications where the eye must be protected against intense glare and radiation.

The construction comprises an eye piece formed as a neutral density attenuator (2) overlayed by a layer of transparent synthetic plastics material (3) capable of modifying the radiation transmission characteristics of the neutral density attenuator. By utilising neutral density attentuators there is a reduction of visible radiation without any alteration in spectral distribution.

## EYE PROTECTION MEANS

This invention relates to eye protection means.

There are numerous industrial applications where the eyes of an operative must be protected from the effects of high intensity glare. Thus in welding and in hot metal and glass applications (melting, casting, etc.) the light emitted has an intensity sufficient to cause painful eye discomfort and possibly permanent damage if the torch flame or the hot metal is viewed with the naked eye. At the same time, the operative must be allowed to observe the area of the weld taking place, and observe the surface of molten metal to allow the correct performance of the functions required.

This has led to the widespread use of glasses, goggles and shields provided with light filters of various colours and degrees of density. However, the filters presently employed, blue, yellow, and most commonly, green, effectively prevent the wearer from perceiving colour in the visible spectrum range of electromagnetic energy (visible light), i.e., the wearer is totally blind to colours other than that of the filters.

With filters in the denser ranges, there is the added disadvantage that the attenuation of illuminance in normal room lighting is so reduced that the wearer has to remove the eye protection means to adjust furnace or torch flame controls, set the casting machine etc. These difficulties are at their worst when having preheated an alloy prior to casting (wearing eye protection) the technician has to move to the furnace, open the furnace door, remove the hot mould and place this in the casting machine, and then immediately reheat the alloy and cast. In this cycle there are several high and low levels of light intensities to be accommodated in a very short time, and removal and replacement of the eye protection means is highly dangerous as well as inconvenient. Also such means as furnaceman's glasses and welding goggles are designed for observing relatively static heat sources and to resist sparks. They do not afford protection from metal splash or from casting mould fragments ejected from, e.g., a centrifugal casting machine. Although rare, these events can occur with great force.

The object of the present invention is to provide eye protection means which avoid the above disadvantages, and provide protection against harmful ultra violet and infra-red radiation.

According to the present invention, eye protection means comprise an eye piece or eye pieces formed as neutral density attenuators, overlaid by a layer of transparent synthetic plastics material capable of modifying the radiation transmission characteristics of the neutral density attenuators.

Preferably, the synthetic plastics layer is of polycarbonate. By utilising neutral density attenuators there is the reduction of visible radiation without any alteration in spectral distribution, normal colour balance and its ability to be perceived being preserved regardless of the degree of filtration, a most desirable feature. In certain applications such as metal melting and casting, particularly when handling noble metals such as gold, so-called "colour temperature" is a most important temperature control guide.

The attenuators of the invention can be made of a shape and thickness to suit conventional spectical frames, protective goggles and face screens or visors.

The attenuators may be used with a uniform density or with a progressive change in transmission from top to bottom, side to side, from periphery to centre and/or in any combination or range of transmissions to enable the wearer to view peripheral or ancilliary objects or events in normal room lighting and then to view the heat/light radiation source without adjustment or removal of eye protection.

By providing an overlay of e.g. polycarbonate, provision is made for any risk of impact from molten metal or other missiles or from chemical splash. Clear polycarbonate covers would be incorporated with, or bonded to the attenuators to provide protection at least to British Standard minimum requirements for industrial impact protection. Where such protection is not required, the attenuators may be made-up as "clip-overs" for conventional prescription glasses or as a filter "inset" into otherwise clear glass/plastic glasses or prescription eye lenses.

Most surprisingly, it has been found that an overlay of, e.g. clear polycarbonate, whilst not affecting spectral distribution, modifies the radiation transmission properties of the neutral density attenuators, by unexpectedly reducing particularly the transmission of infra red radiation. Not only that, there is also the elimination of any transmission of harmful ultra violet radiation. Both aspects are greatly to the benefit of the user, leaving the user protected against harmful glare and radiation, and yet with clear vision, and with the additional protection against damage from any flying debris afforded by the overlay.

One embodiment of the invention will now be described with reference to the accompanying drawings, in which:-

Figure 1 is a sectional side elevation through part of eye protection means in accordance with the invention; and

Figure 2 is a graphical representation of comparative testing of the radiation transmission characteristics of eye protection means in accordance with the invention with other eye protection means.

In the drawings, eye protection means 1 in the form of a lens for a pair of protective goggles comprises a neutral density attenuator 2 having a thickness of 0.1mm sandwiched between two polycarbonate discs 3 each of a thickness of approximately 1.5mm. The polycarbonate discs can be bonded to the neutral density attenuator by any conventional means such as by heat.

An eye protection means as illustrated in

- 6 -

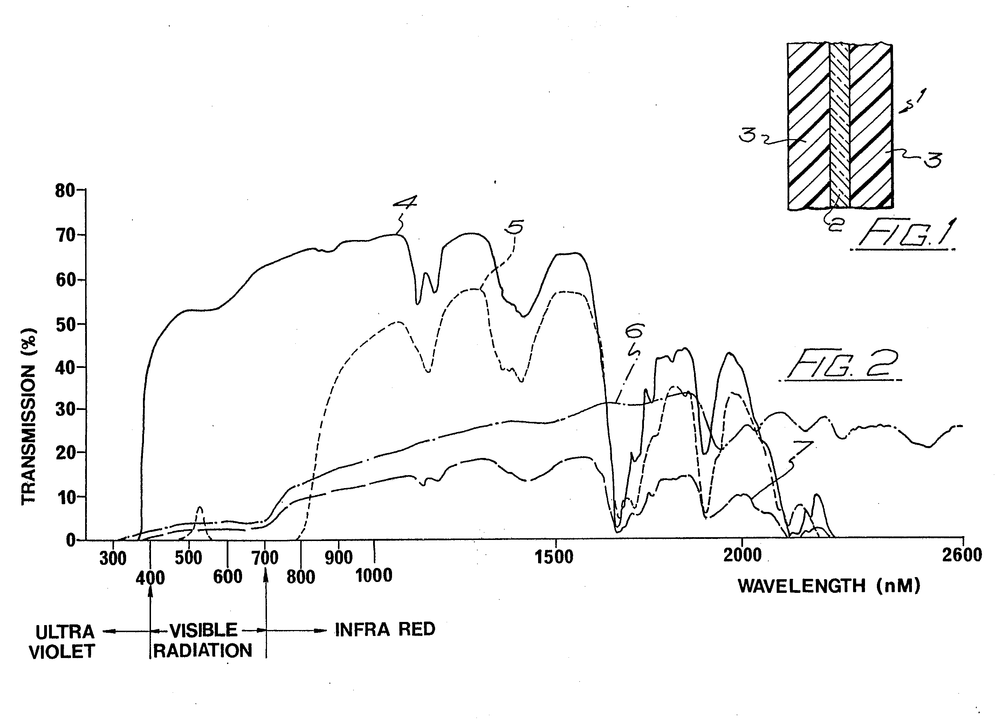

Figure 1 was placed in the sample tray of a Perkin Elmer 330 spectrophotometer and the radiation transmission characteristics obtained and reproduced in visual form via a pen recorder. For comparison purposes three superimposed discs of polycarbonate, a neutral density filter alone and a conventional green welding goggle filter (No. 5 GWF) in accordance with BS679: 1959 was similarly tracked on the spectrophotometer.

The results of these tests are shown in the graphs of Figure 2, where line 4 is the transmission characteristics of the polycarbonate discs, line 5 those of a standard welding goggle filter, line 6 of the neutral density filter alone and line 7 of the eye protection means in accordance with the invention.

As the graphical representations show, the standard welding goggle filter substantially eliminates any transmitted radiation in the visible radiation range other than the colour green. As a result of this, whilst the eye is protected when viewing any intense source of radiation such as a welding flame, there is a total absence of colour appreciation and which, in certain operations is highly disadvantageous. In contrast with this a neutral density attenuator alone provides a substantially constant transmission of radiation across the whole of the visible radiation range and accordingly whilst such an attenuator gives ample protection to the eye when effecting any operation such as viewing hot metal, glass or a welding flame, there is sufficient radiation transmission to allow full colour appreciation because of the absence of an alteration in spectral distribution. With the standard welding goggle there is considerable transmission in the infra-red range filter and with the neutral density attenuator there is transmitted radiation in the ultra violet range, both of which can be harmful to the eye.

When considering the transmission characteristics of polycarbonate discs there is no transmission in the harmful ultra violet range and virtually no reduction in transmission over the whole of the visible radiation range, but a substantial transmission in the infra red radiation range.

The surprising effect of the invention is shown by the graph representing the transmission characteristics of the eye protection means where a neutral density attenuator is combined with polycarbonate. In the first place there is the substantial elimination of any transmission in the

- 8 -

harmful ultra violet range, and transmission in the visible radiation range at a level most similar to that of a neutral density attenuator alone. There is however in the infra red range a noticeable reduction in the transmission of infra red radiation, along with a positive cut-off of infra red radiation at approximately 2250 nanometres.

As has been mentioned earlier the additional advantage of an eye piece in accordance with the invention is the strength characteristic inherent in polycarbonate, and when the criteria of BS 2092 are met and the user is amply protected against damage to the eye from any splashed metal or other flying fragments.

CLAIMS

1. Eye protection means comprising an eye piece or eye pieces formed as neutral density attenuators overlayed by a layer of transparent synthetic plastics material capable of modifying the radiation transmission characteristics of the neutral density attenuators.

2. Eye protection means as in Claim 1, wherein the synthetic plastics material is polycarbonate.

3. Eye protection means as in Claim 2, wherein a neutral density attenuator is sandwiched between two layers of polycarbonate.

4. Eye protection means as in Claim 2 or Claim 3,wherein the polycarbonate layers and the neutral density attenuator are bonded together.

5. Eye protection means as in any of Claims 1 to 4, wherein the neutral density attenuators have a uniform density across their width and height.

6. Eye protection means as in any of Claims 1 to 4, wherein the neutral density attenuators have a progressive change in transmission characteristics from top to bottom, from side to side, or from periphery to centre, in any combination or range of transmissions.

7. Eye protection means as in any of Claims 1 to 6, and in the form of a lens for conventional spectacle frames, protective goggles, or face screens or visors.

8. Eye protection means as in any of Claims 1 to 6, in the form of clip-overs for conventional prescription glasses.

9. Eye protection means as in any of Claims 1 to 6, in the form of an inset into otherwise clear glass/plastic glasses or prescription eye lenses.

10. Eye protection means substantially as hereinbefore described with reference to the accompanying drawings.

FIG.1

FIG.2

TRANSMISSION (%)

80
70
60
50
40
30
20
10
0

300   400   500   600   700   800   900   1000        1500        2000        2600

WAVELENGTH (nM)

ULTRA VIOLET ← → VISIBLE RADIATION ← → INFRA RED

0115656

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| Y,A | DD-A- 89 493 (KRAUSE et al.) <br> * Claims 1-3; figures 1-3; column 2, line 19 - column 3, line 63 * | 1,4,6- 8 | A 61 F 9/06 <br> G 02 B 5/22 <br> G 02 C 7/10 |
| Y | DD-A- 89 736 (KRAUSE et al.) <br> * Claim 1; figures 1,2 * | 1 | |
| Y,A | US-A-3 382 183 (DONOIAN et al.) <br> * Claim 1; column 5, lines 9-49 * | 1,2 | |
| Y | US-A-3 112 490 (MALCOM, JR.) <br> * Claim 1 * | 1 | |
| A | DE-B-2 440 468 (DEUTSCHE SPIEGELGLAS AG) <br> * Claims 1,5 * | 2 | **TECHNICAL FIELDS SEARCHED (Int. Cl. ³)** |
| A | US-A-4 200 360 (MUTZHAS) | | A 61 F 9/00 <br> G 02 B 5/00 <br> G 02 C 7/00 |
| A | CH-A- 414 894 (HAMBACH) | | |
| A | DE-B-2 658 376 (AGA AB) | | |

The present search report has been drawn up for all claims

| Place of search | Date of completion of the search | Examiner |
|---|---|---|
| BERLIN | 30-03-1984 | KANAL P K |